(19) Europäisches Patentamt
European Patent Office
Office européen des brevets

(11) Veröffentlichungsnummer : **0 453 944 B1**

(12) # EUROPÄISCHE PATENTSCHRIFT

(45) Veröffentlichungstag der Patentschrift : **16.03.94 Patentblatt 94/11**

(51) Int. Cl.$^5$ : **C07C 17/02,** C07C 19/045

(21) Anmeldenummer : **91106153.9**

(22) Anmeldetag : **17.04.91**

(54) **Katalysatorsystem und Verfahren zur Herstellung von 1,2-Dichlorethan durch Chlorierung von Ethylen unter Einsatz dieses Katalysatorsystems.**

(30) Priorität : **19.04.90 DE 4012529**

(43) Veröffentlichungstag der Anmeldung : **30.10.91 Patentblatt 91/44**

(45) Bekanntmachung des Hinweises auf die Patenterteilung : **16.03.94 Patentblatt 94/11**

(84) Benannte Vertragsstaaten : **BE DE FR GB IT NL SE**

(56) Entgegenhaltungen :
**EP-A- 0 112 544**
**EP-A- 0 146 882**
**DE-A- 2 649 533**
**US-A- 4 410 747**

(73) Patentinhaber : **WACKER-CHEMIE GMBH**
**Hanns-Seidel-Platz 4**
**D-81737 München (DE)**

(72) Erfinder : **Schmidhammer, Ludwig, Dr.**
**Pappelweg 5**
**W-8261 Haiming (DE)**
Erfinder : **Haselwarter, Klaus**
**Döblstrasse 3**
**W-8261 Emmerting (DE)**
Erfinder : **Klaus, Herrmann**
**Adalbert-Stifter-Strasse 1 a**
**W-8261 Marktl (DE)**
Erfinder : **Dummer, Gerhard**
**Immanuel-Kant-Strasse 65**
**W-8263 Burghausen (DE)**
Erfinder : **Mohr, Klaus-Peter**
**Immanuel-Kant-Strasse 44**
**W-8263 Burghausen (DE)**

**EP 0 453 944 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Katalysatorsystem zur Herstellung von 1,2-Dichlorethan durch Reaktion zwischen Ethylen und Chlor, gegebenenfalls in Gegenwart eines Inhibitors zur Verringerung der Nebenproduktbildung, sowie ein Verfahren zur Herstellung von 1,2-Dichlorethan unter Einsatz dieses Katalysatorsystems.

Die Herstellung von 1,2-Dichlorethan (EDC) durch Reaktion von Ethylen mit Chlor in 1,2-Dichlorethan als Lösungsmittel und Reaktionsmedium ist bekannt. Zur Beschleunigung der Chloraddition an die Ethylenmolekel verwendet man als Katalysatoren neben den Chloriden der Elemente der 3. bis 6. Hauptgruppe bzw. der 1., 4. und 6. Nebengruppe des Periodensystems vor allem wasserfreies Eisen-III-Chlorid, da letzteres leicht zugänglich und preisgünstig ist (CA-A 689991, DE-C 640827, DE-B 1768367). Als hauptsächliche Nebenprodukte fallen bei dieser Reaktion Ethylchlorid, durch konkurrierende Hydrochlorierung von Ethylen, und unter Chlorwasserstoffentwicklung 1,1,2-Trichlorethan als Folge von einer durch Ethylen induzierten Substitution von gebildetem EDC an.

Vielfach wird die Ethylenchlorierung auch in Gegenwart von Sauerstoff als Substitutionsinhibitor durchgeführt (US-A 2601322, DE-A 1568583).

Die Addition von Chlor an Ethylen wird in der Technik sowohl bei Reaktionstemperaturen um den atmosphärischen Siedepunkt von EDC durchgeführt, wobei die freigesetzte Reaktionswärme zum Abdestillieren und rektifizierenden Reinigen des Reaktionsproduktes und gegebenenfalls auch von Roh-EDC anderer Proveniencen ausgenutzt wird, als auch bei tieferen Temperaturen von 30 bis 60°C durchgeführt, wie in Ullmanns Encyclopädie der technischen Chemie, Band 9, Seite 427 (1975) beschrieben. In letzterem Fall ist das Temperaturniveau der Reaktion jedoch so niedrig, daß die freigesetzte Reaktionsenthalpie nicht sinnvoll ausgenutzt werden kann, sondern durch Umpumpen des Reaktionsmediums über Wärmetauscher ins Kühlwasser oder an die Luft abgeführt werden muß (DE-A 1905517).

Das bei tieferen Temperaturen hergestellte rohe, katalysatorhaltige EDC wird üblicherweise aus dem Reaktor flüssig abgezogen und muß zwecks Katalysatorentfernung mit Sauerwasser und anschließend mit wäßrigen Alkalilösungen gewaschen werden, um das Rohprodukt zu neutralisieren. Nach Phasentrennung durch Dekantieren und Abtrennen der wäßrigen Schicht ist das verbleibende wassergesättigte Roh-EDC anschließend in bekannter Weise, aber aufwendig, destillativ aufzuarbeiten.

Gemäß der DE-A 2427045 chloriert man Ethylen bei Temperaturen von 100 bis 130°C und entsprechend hohen Drucken, um die Reaktion in der Flüssigphase durchführen zu können, in Gegenwart von Eisen-III-Chlorid als Katalysator und führt das gebildete mit dem umlaufenden Reaktionsmedium zu einer unter niedrigerem Druck stehenden Zone, in der das gebildete Roh-EDC durch die bei der Umsetzung von Chlor mit Ethylen freiwerdende Reaktionswärme verdampft und unter Rückführung von hochsiedenden Verunreinigungen in die Reaktionszone rektifiziert wird. Dadurch kommt es zu einer Hochsiederanreicherung im Reaktionskreislauf. Durch batchweises Abziehen von flüssigem katalysatorhaltigem Kreislaufprodukt aus den Sumpf des Reaktors kann zwar diese Anreicherung in den gewünschten Grenzen gehalten werden; um eine Katalysatorverarmung zu verhindern, muß jedoch von Zeit zu Zeit Eisen-III-Chlorid in entsprechenden Mengen zum Reaktionsmedium zugesetzt werden. Schwierigkeiten bereitet darüberhinaus auch die Entsorgung des Reaktorsumpfabzuges. Daneben ist auch die Verwendung von Eisen-III-Chlorid als Katalysator bei höheren Reaktionstemperaturen mit gewissen Nachteilen verbunden. Einmal fördert Eisen-III-Chlorid mit zunehmender Temperatur die Zersetzung des gebildeten EDC's unter Abscheidung teerartiger, amorpher und kohlenstoffreicher Ablagerungen (J. Soc. Chem. Ind. <u>69</u> (1950) Seite 289), wodurch neben einer mit steigender Temperatur zunehmenden Nebenproduktbildung vor allem auch die Ethylenausbeute stark zurückgeht. Zum anderen wirkt Eisen-III-Chlorid in Gegenwart von Spuren Wasser gegenüber den üblicherweise beim Reaktor- und Apparatebau verwendeten Werkstoffen korrosiv, wobei die Geschwindigkeitsrate des Korrosionsangriffes ganz allgemein mit steigender Temperatur überproportional zunimmt.

Das korrosive Verhalten von Eisen-III-Chlorid wird durch Chlorwasserstoff, der aufgrund unerwünschter Nebenreaktionen praktisch immer zugegen ist, noch potenziert, weil sich dadurch der stark korrosive Komplex Wasserstofftetrachloroferrat bildet, der sehr leicht aggressive Protonen abgibt. Aus diesem Grund hat man versucht, in Abwesenheit von Metallsalzkatalysatoren, durch Zugabe organischer Katalysatoren, wie zum Beispiel Hydroxylgruppen enthaltender Aromaten, die Reaktion zwischen Ethylen und Chlor komplett und selektiv zu gestalten (DE-B 1902843). Dies gelingt jedoch nur kurzzeitig, weil vor allem bei erhöhten Temperaturen mit der Zeit eine Chlorierung des Aromatenkerns eintritt und andererseits mit steigendem Chlorierungsgrad die katalytische Wirksamkeit solcher organischen Katalysatoren drastisch zurückgeht.

Nach dem Verfahren der EP-A 82342 (Eisen-III-Chlorid-Katalysator im Gegenwart von Stickstoffbasen, wie Ammoniak, Aminen, oder Salzen dieser Basen) bzw. der EP-A 111203 (Alkali- oder Erdalkali-Tetrachloroferrat-Katalysatoren) kann vor allem im mittleren Temperaturbereich von 90 bis 120°C die durch Eisen-III-Chlorid als Katalysator bei der EDC-Herstellung verursachte Korrosion in Reaktoren, die mit herkömmlichen metallischen Werkstoffen gefertigt worden sind, erheblich vermindert werden, wenn man den Eisen-III-Chlorid-Katalysator mit den dort genannten Zusätzen beaufschlagt. Daneben wirken sich diese Zusätze auch vorteilhaft auf die Nebenproduktbildung aus, die dadurch verringert wird. Bei erhöhten Reaktionstemperaturen, die für eine wirtschaftliche Ausnutzung der bei der Ethylenchlorierung freiwerdenden Reaktionsenthalpie unerläßlich sind, gehen diese korrosionsinhibierenden und die Reaktionsselektivität fördernden Effekte jedoch deutlich zurück, weil die Ammonium- bzw. Alkali- oder Erdalkalitetrachloroferrate, die sich durch Zusatz von Ammoniumchlorid oder organischen Aminhydrochloriden bzw. von Alkali- oder Erdalkalichloriden zu Eisen-III-Chlorid bilden, thermisch labil sind und mit steigender Temperatur immer mehr in die Ausgangskomponenten zerfallen und somit daraus wiederum äußerst korrosives Wasserstofftetrachloroferrat vermehrt neben den, nunmehr separat vorhandenen und isoliert praktisch inerten, Ammonium- bzw. Alkali- oder Erdalkalichloriden entsteht. Im Falle der Ammonium- bzw. Aminhydrotetrachloroferratkomplexe kommt noch verstärkend hinzu, daß die Ammoniumionen noch Brönsted-Säuren sind, die mit steigender Temperatur unter Ausbildung von Aminbasen hochkorrosive Protonen abspalten.

Die EP-A 113287 lehrt ein Verfahren, wonach in Gegenwart von Eisen-III-Chlorid bei Temperaturen von 140 bis 180°C etwa 85 % der bei der Ethylenchlorierung freiwerdenden Reaktionswärme zur Dampferzeugung genutzt werden kann. Dabei wird das die Reaktionszone verlassende EDC vor der weiteren Aufarbeitung bzw. Rückführung in die Reaktionszone durch Wärmetausch mit Wasser abgekühlt. Abgesehen von dem negativen Korrosionsverhalten von blankem Eisen-III-Chlorid, entstehen dabei jedoch auch viele Nebenprodukte. Sicherlich ist es jedoch der technisch richtigere Weg, die freigesetzte Reaktionsenthalpie zur Erzeugung von Dampf bzw. zum Aufheizen eines Wärmeträgermediums auszunutzen, weil man dadurch flexibler wird und auch bei im Tagesgeschehen auftretenden bzw. generell "unbalanced"-Verhältnissen eine optimale Rückgewinnung der Reaktionsenthalpie der Ethylenchlorierung erzielt werden kann.

Bei dem verfahren gemäß der EP-A 75742 wird das Reaktionsgemisch aus der Ethylenchlorierung in 2 Teilströme aufgeteilt, von denen einer über einen Wärmetauscher geleitet wird und dann in den Kreislauf zurückkehrt, während der zweite Teilstrom entspannt und einer Rektifizierkolonne zugeführt wird, welche über den genannten Wärmetauscher beheizt wird. Bei dieser Verfahrensweise ist die eben diskutierte Flexibilität nicht gegeben, sodaß bei Abweichungen vom "balanced process", aus Mangel an ausreichend freizusetzender Reaktionswärme, oder auf Grund einer über den "balanced process" hinaus erhöhten EDC-Produktion, die in das Reaktionssystem integrierte Schwersiederkolonne zusätzlich mit Dampf beheizt bzw. im umgekehrten Fall überschüssige Reaktionsenthalpie über einen Trimmkühler ins Kühlwasser bzw. an die Luft abgeführt werden muß. Abweichungen vom "balanced process" treten beispielsweise dann auf, wenn wegen temporären Chlormangels die Ethylenchlorierung zurückgefahren werden muß oder durch ein Überangebot an externem Chlorwasserstoff die Oxychlorierung von Ethylen höher fährt oder bei normaler Fahrweise der Direktchlorierung die Oxichlorierung, wegen reduzierter Vinylchloridmonomer-Produktion, auch niedriger fährt.

Es bestand daher die Aufgabe, ein Katalysatorsystem zur Herstellung von 1,2-Dichlorethan zu entwickeln, das in allen technisch wichtigen Temperaturbereichen, das heißt von 0°C bis 300°C, bei der Chlorierung von Ethylen ein Optimum an Selektivität bzw. Ausbeute, sowie ein Minimum an Korrosion gewährleistet.
Weiter bestand die Aufgabe dieses Katalysatorsystem auf ein einfaches und wirtschaftliches Verfahren zur Herstellung von EDC durch Chlorierung von Ethylen zu übertragen, das je nach den lokalen Gegebenheiten ermöglicht, entweder zumindest einen Teil der freiwerdenden Reaktionsenthalpie zur Reingewinnung von gebildetem EDC in Spaltqualität zu verwenden oder praktisch die gesamte freigesetzte Reaktionsenthalpie zur Erzeugung von Dampf mit einem technisch sinnvollen Druck auszunutzen, wobei das Katalysatorsystem im Reaktionsmedium verbleiben soll, und das gebildete EDC vor dem Einsatz in einem Pyrolyseofen nur noch von geringen Mengen an hochsiedenden Verunreinigungen befreit werden muß.

Gegenstand der Erfindung ist ein Katalysatorsystem zur Herstellung von 1,2-Dichlorethan durch Reaktion zwischen Ethylen und Chlor in einem Lösungsmittel, gegebenenfalls in Gegenwart eines Inhibitors zur Verringerung der Nebenproduktbildung, bestehend aus einem Phenolatchlorokomplex der Formel

$$Me^{+n}[Z^{+m}Cl_m \cdot L]_n$$

worin

- n — eine ganze Zahl von 1 bis 3 ist,
- m — eine ganze Zahl von 1 bis 6 ist,
- Cl — ein Chloridanion bedeutet,
- $Me^+$ — ein Wasserstoffproton und/oder Metallkation der Elemente der 1. und/oder 2. Hauptgruppe oder der Lanthaniden-Gruppe des Periodensystems der Elemente (PSE) bedeutet,
- $Z^+$ — ein Metallkation der Elemente der 3., 4., 5. oder 6. Hauptgruppe oder der 1., 4., 6. oder 8. Nebengruppe des PSE bedeutet, und
- L — ein Phenolatanion ist der Formel:

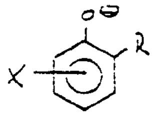 und/oder 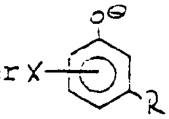 und/oder 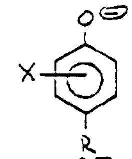 und/oder 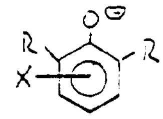

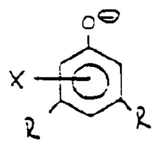 und/oder 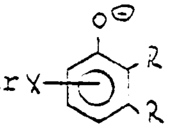 und/oder 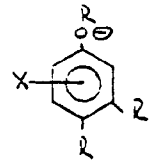 und/oder 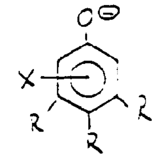

worin

X 0 bis 4 Chloratome bedeutet und

R ein Wasserstoffatom, und/oder eine Hydroxylgruppe, und/oder ein Halogen, und/oder ein linearer oder verzweigter Alkyl- oder Chloralkylrest mit 1 bis 6 C-Atomen, und/oder ein Alkoxy- oder Chloralkoxyrest mit 1 bis 6 6-Atomen in linearer oder verzweigter Anordnung, ist.

Das erfindungsgemäße Katalysatorsystem wird durch Zusammenmischen der Komponente $Me^{+n}Cl_n$, das heißt von Chlorwasserstoffgas und/oder Alkalichloriden und/oder Erdalkalichloriden oder von Chloriden der Lanthaniden mit der Komponente $Z^{+m}Cl_m$, das heißt mit Metallchloriden der Elemente der 3., 4., 5. oder 6. Hauptgruppe oder der 1., 4., 6. oder 8. Nebengruppe des Periodensystems der Elemente, sowie mit den phenolischen Verbindungen der oben beschriebenen allgemeinen Formeln hergestellt.

Die Zumischung erfolgt in jeweils nahezu äquimolaren Mengen von $Z^{+m}Cl_m$ und phenolischer Verbindung zueinander, mit einem Streubereich von jeweils 0.9 bis 1.1 : 1 äquivalenten Anteilen, als Lösung oder Suspension, vorzugsweise in 1,2-Dichlorethan. Die Äquivalenz zwischen $Me^{+n}Cl_n$ und phenolischer Verbindung kann auch einen Streubereich von jeweils 0.5 bis 1.5 : 1 aufweisen, vorzugsweise beträgt der Streubereich ebenfalls 0.9 bis 1.1 : 1.

Die Herstellung der erfindungsgemäßen Katalysatoren $Me^{+n}[Z^{+m}Cl_m \cdot L]_n$ aus dem Gemisch von $Z^{+m}Cl_m$, $Me^{+n}Cl_n$ und der phenolischen Verbindung kann in einem separaten Reaktionsschritt durch Mischen und gegebenenfalls mehrstündiges Erhitzen unter Rückfluß erfolgen oder in situ in der Reaktionszone des Ethylenchlorierungsreaktors, nach Zugabe des Gemisches zum Reaktionsmedium.

Bei chlorierten phenolischen Liganden kann die Kernchlorierung dabei vor dem Zusammenmischen mit den übrigen Bestandteilen des Katalysatorsystems in der EDC-Lösung separat oder in einem separaten Katalysatoransatzrührwerk erfolgen. Vorzugsweise erfolgt die Kernchlorierung in situ in der Reaktionszone des Ethylenchlorierungsreaktors, nach Zugabe des Gemisches der einzelnen Katalysatorkomponenten.

Als Alkali- und Erdalkalichloride werden vorzugsweise Natriumchlorid oder Calciumchlorid eingesetzt. Von den Lanthaniden wird vorzugsweise Cer-III-Chlorid verwendet. Die das Zentralatom Z des Phenolatchlorokomplexes liefernden Metallchloride sind vorzugsweise Aluminiumchlorid, Zinn-IV-Chlorid, Wismut-III-Chlorid, Tellur-IV-Chlorid, Gold-III-Chlorid, Titan-IV-Chlorid, Wolfram-VI-Chlorid, Thallium-I-chlorid und Eisen-III-Chlorid. Besonders bevorzugt wird Eisen-III-Chlorid eingesetzt. Als phenolische Verbindungen eignen sich besonders Phenol; ortho-, meta-, para-Kresol; ortho-, meta-, para-Chlorphenol; 2-sek-Butylphenol, Brenzcatechin, Resorcin, Hydrochinon, Guajakol, Pyrogallol, Oxyhydrochinon, Phloroglucin und 3,5-Ditert.-Butyl-Hydrochinon.

Besonders bevorzugte Katalysatoren sind Wasserstoff-metakresolat-trichloroferrat, Natrium-ortho-kresolat-trichloroferrat, Calcium-bis-para-kresolat-trichloroaluminat und Certris-ortho-kresolat-trichloroaluminat, die gegebenenfalls einfach oder mehrfach kernchloriert sein können.

Ein weiterer Gegenstand der Erfindung ist ein Verfahren zur Herstellung von 1,2-Dichlorethan durch Reaktion von Ethylen mit Chlor, in einer Reaktionszone, die ein im Kreis umlaufendes flüssiges Medium, bestehend aus Chlorkohlenwasserstoffen mit 2 Kohlenstoffatomen, enthält, bei einer Temperatur unterhalb der Verdampfungstemperatur des Mediums, bei dem in der Reaktionszone herrschendem Druck, gegebenfalls in Gegenwart von Sauerstoff als Inhibitor zur Vermeidung von Nebenreaktionen, dadurch gekennzeichnet, daß

a) die Konzentration des im Reaktionsmedium gelösten oder suspendierten Katalysatorsystems $Me^{+n}[Z^{+m}Cl_m \cdot L]_n$, berechnet als $Z^{+m}Cl_m$ 0.01 bis 1.0 Gew%, bezogen auf die Menge an Reaktionsmedium, beträgt und das Katalysatorsystem, unter Ersatz des verbrauchten Ethylen und Chlor im Kreis geführt wird, wobei man

b) bei Reaktionstemperaturen von 0 bis 300°C und bei Drükken, die ein Sieden des Reaktionsmediums im Reaktionsraum verhindern, unter teilweiser bis vollständiger Ausnutzung der bei der Ethylenchlorierung freiwerdenden Reaktionsenthalpie, zur Erzeugung von 1,2-Dichlorethan-Dampf, dampfförmiges 1,2-Dichlorethan aus der Reaktionszone in eine Zone niedrigeren Druckes abzieht und diese Dämpfe dabei in

den Sumpf einer Rektifizierkolonne einleitet, und

c) bei Reaktionstemperaturen von 0 bis 120°C, die Brüden am Kolonnenkopf durch Kühlen mit Wasser oder Luft kondensiert, bei Reaktionstemperaturen von 120 bis 300°C die Kondensationswärme der Brüden , durch Wärmetausch der Brüden der Rektifizierkolonne mit einem Wärmetauschmedium ausnutzt, und

d) unter Aufrechterhaltung eines Mindestrücklaufverhältnisses, ausgedrückt als Gewichtsverhältnis Rücklauf zu erzeugtem Produkt, bei der Niedertemperaturfahrweise von 2:1 Gewichtsteilen und bei der Hochtemperaturfahrweise von 1:1 Gewichtsteilen, die kondensierten Brüden bei der Niedertemperaturfahrweise mehr oder weniger stark unterkühlt, bei der Hochtemperaturfahrweise jedoch um maximal 5°C unterkühlt, bezogen auf die Siede- oder Kondensationstemperatur, in den Reaktionsraum zurückführt, und

e) im Sumpf der Rektifizierkolonne das erzeugte 1,2-Dichlorethan in flüssiger Form entnimmt.

Als Reaktoren für die Ethylenchlorierung eignen sich Schlaufenreaktoren, wie sie beispielsweise in der DE-A 2427045 beschrieben sind, oder Schleifenreaktoren gemäß DE-B 1768367 oder gemäß beiliegender Figur 1, oder Turmreaktoren gemäß beiliegender Figur 2. Die Chlorierung wird in einem, im Kreis umlaufenden flüssigen Medium, bestehend aus Chlorkohlenwasserstoffen mit 2 Kohlenstoffatomen durchgeführt. Vorzugsweise wird als Reaktionsmedium 1,2-Dichlorethan eingesetzt. Die Geschwindigkeit des im Kreis umgewälzten Reaktionsmediums liegt vorzugsweise bei Werten zwischen 0.1 und 5 m/s. Der Umlauf des flüssigen Reaktionsmediums im Reaktor kann beispielsweise mittels Pumpe und/oder nach dem Thermosiphon- bzw. Mammutpumpenprinzip bewirkt werden.

Der Reaktor wird mit Ethylen und Chlor beschickt, wobei das molare Verhältnis von Ethylen zu Chlor vorzugsweise zwischen 1 : 1 und 1.005 : 1 beträgt. Die Gasgeschwindigkeit im Reaktionsraum beträgt vorzugsweise 0.01 bis 1 m/s, bezogen auf den Ethylengasstrom.

Die Konzentration des im Reaktionsmedium gelösten oder suspendierten Katalysatorsystems $Me^{+n}[Z^{+m}Cl_m \cdot L]_n$ beträgt zwischen 0.01 und 1.0 Gew% vorzugsweise 0.2 bis 0.7 Gew%, jeweils bezogen auf die Menge an Reaktionsmedium und berechnet als $Z^{+m}Cl_m$. In einer bevorzugten Ausführungsform wird das Katalysatorsystem in Form der Einzelkomponenten im Reaktionsmedium gelöst oder suspendiert, wobei die Einzelkomponenten vorzugsweise in einem molaren Verhältnis von 0.9 bis 1.1 : 1 zueinander eingesetzt werden.

Besonders bevorzugte Katalysatoren sind Wasserstoff-metakresolat-trichloroferrat, Natrium-ortho-kresolat-trichloroferrat, Calcium-bis-para-kresolat-trichloroaluminat und Certris-ortho-kresolat-trichloroaluminat, die gegebenenfalls einfach oder mehrfach kernchloriert sein können.

Insbesonders wird Natrium-ortho-kresolat-trichloro-ferrat, vorzugsweise in einer Konzentration von 0.3 bis 0.5 Gew%, bezogen auf das Reaktionsmedium, als Katalysatorsystem eingesetzt. Es wird vorzugsweise in situ gebildet, nach Zugabe eines, im erwähnten Äquivalenzbereich, äquimolaren Gemisches von NaCl, $FeCl_3$ und o-Kresol zum Reaktionsmedium.

Das Katalysatorsystem wird mit dem Reaktionsmedium unter Ersatz des verbrauchten Ethylen und Chlor im Kreis geführt.

Zur Unterdrückung von Nebenreaktionen kann gegebenenfalls als Inhibitor Sauerstoff oder Luft eingesetzt werden. Die Konzentration beträgt dabei 0.01 bis 10 Vol%, vorzugsweise von 0.8 bis 1.5 Vol%, jeweils als Sauerstoff berechnet und auf die Chlorgasmenge bezogen.

Die mittlere Verweilzeit des Reaktandengemisches liegt bei 0.2 bis 2 Minuten, bezogen auf den leeren Reaktions-, Misch- und Umwälzraum bei Normalbedingungen (0°C, 1013 mbar). Die Reaktionszeit der Reaktanden im Reaktionsraum beträgt vorzugsweise zwischen 1.5 und 60 Sekunden.

Die Chlorierungsreaktion wird bei Reaktionstemperaturen zwischen 0 und 300°C und bei Drücken durchgeführt, die ein Sieden des Reaktionsmediums im Reaktionsraum verhindern. Bei Niedertemperaturfahrweise beträgt die Reaktionstemperatur 0 bis 120°C, vorzugsweise 40 bis 80°C, und der Druck im Reaktionsraum vorzugsweise zwischen 1 und 5 bar absolut. Bei Hochtemperaturfahrweise beträgt die Reaktionstemperatur 120 bis 300°C, vorzugsweise 140 bis 170°C,und der Druck im Reaktionsraum vorzugsweise zwischen 5 und 10 bar absolut.

Das 1,2-Dichlorethan wird aus der Reaktionszone in eine Zone niedrigeren Druckes abgezogen und die dabei entstehenden Dämpfe in eine Rektifizierkolonne eingeleitet. Die Rektifizierkolonne ist vorzugsweise eine gepackte Säule oder eine Bodenkolonne. Die Anzahl der theoretischen Böden beträgt vorzugsweise zwischen 1 und 5.

Bei Niedertemperaturfahrweise der Ethylenchlorierung werden die Brüden am Kopf der Rektifizierkolonne in einem Kondensator durch indirekten Wärmetausch mit Wasser oder Luft kondensiert. Der Druck am Kolonnenkopf beträgt vorzugsweise 0.2 bis 0.9 bar abs.

Eine Teilmenge des Kondensats wird als flüssiger Rücklauf der Rektifizierkolonne aufgegeben, die Restmenge wird mehr oder weniger stark unterkühlt, vorzugsweise mit einer gegenüber der Siedetemperatur um etwa 5 bis 50°C verminderten Temperatur, dem im Kreis geführten Reaktionsmedium zugeführt und in den Reaktionsraum zurückgeführt. Bei der Niedertemperaturfahrweise beträgt das Mindestrücklaufverhältnis, ausgedrückt als Gewichtsverhältnis Rücklauf zu erzeugtem Produkt, vorzugsweise 2:1 Gewichtsteile. Die nicht kondensierbaren Anteile des Kopfprodukts der Rektifizierkolonne werden in die Atmosphäre oder in eine Verbrennungsanlage abgeleitet.

Im Sumpf der Rektifizierkolonne wird das erzeugte 1,2-Dichlorethan entnommen. Die verbliebene Reaktionswärme kann über einen im Umwälzkreis befindlichen Wärmetauscher ins Kühlwasser oder in die Luft abgeführt werden.

Bei Hochtemperaturfahrweise der Ethylenchlorierung werden die Brüden am Kopf der Rektifizierkolonne in einem Wärmetauscher kondensiert. Der Druck am Kolonnenkopf beträgt vorzugsweise 4.5 bis 8.3 bar abs..Als Wärmetauschmedien sind beliebige Wärmeträger denkbar, beispielsweise Mineralöle, welche die dabei adsorbierte Wärme an anderer Stelle abgeben und dementsprechend abgekühlt wieder zum Wärmetauscher zurückgeführt werden. Vorzugsweise wird die Kondensationswärme der Brüden zur Erzeugung von Sattdampf mit einem Druck von 2.25 bis 6.0 bar abs., durch Wärmetausch der Brüden der Rektifizierkolonne mit heißem Wasser entsprechenden Druckes, ausgenutzt.

Eine Teilmenge des Kondensats wird als flüssiger Rücklauf der Rektifizierkolonne aufgegeben, die Restmenge wird dem im Kreis geführten Reaktionsmedium zugeführt und in den Reaktionsraum zurückgeführt, wobei die Temperatur des in den Reaktionsraum zurückgeführten Brüdenkondensats um maximal 5° C unterkühlt sein sollte, bezogen auf die Siede- oder Kondensationstemperatur. Bei der Hochtemperaturfahrweise beträgt das Mindestrücklaufverhältnis, ausgedrückt als Gewichtsverhältnis Rücklauf zu erzeugtem Produkt, vorzugsweise 1:1 Gewichtsteile. Die nicht kondensierbaren Anteile des Kopfprodukts der Rektifizierkolonne werden in die Atmosphäre oder in eine Verbrennungsanlage abgeleitet. Im Sumpf der Rektifizierkolonne wird das erzeugte 1,2-Dichlorethan entnommen.

Mit dem erfindungsgemäßen Verfahren kann bei der Niedertemperaturfahrweise die bei der Ethylenchlorierung freigewordene Reaktionsenthalpie bei der Erzeugung des dampfförmigen 1,2-Dichlorethans durch Entspannen des Reaktionsprodukts der Ethylenchlorierung, zu mindestens 40 % ausgenutzt werden.

Bei der erfindungsgemäßen Hochtemperaturfahrweise wird die Rückgewinnung der gesamten bei der Ethylenchlorierung freigewordenen Reaktionsenthalpie möglich. Die Wärmemenge der Reaktion wird gänzlich zur Erzeugung des dampfförmigen 1,2-Dichlorethans durch Entspannung des Reaktionsprodukts ausgenutzt und kann als Kondensationswärme der Brüden durch Wärmetausch zurückgewonnen werden.

Das erfindungsgemäße Verfahren und eine Vorrichtung zur Ethylenchlorierung in der Niedertemperaturfahrweise sind in Fig. 1 und Bsp. 5 exemplarisch dargestellt. In Fig. 2 und Bsp. 6 sind das erfindungsgemäße Verfahren und eine Vorrichtung für die Hochtemperaturfahrweise exemplarisch dargestellt.

Gegenüber den aus dem Stand der Technik bekannten Verfahrensweisen bietet das erfindungsgemäße Verfahren eine Reihe von Vorteilen:

Aufgrund der Entfernung des Reaktionsprodukts aus dem Reaktionsraum in Dampfform entfällt die Notwendigkeit der Katalysatornachdosierung und der Katalysatorentfernung aus dem Rohprodukt. Darauf basiert auch der Wegfall der umweltgerechten Vorbehandlung des durch EDC kontaminierten Waschwassers (z.B. CKW-Entfernung durch Dampfstrippen).

Mit dem Verfahren erhält man hohe Ausbeuten bei extrem niedriger Nebenproduktbildung (hohe Selektivität).

Weil das erfindungsgemäße Katalysatorsystem nahezu nicht korrosiv ist, dadurch keine exotischen Werkstoffe erforderlich sind, und darüberhinaus die Reaktionsapparatur relativ einfach und wenig umfangreich ist, sind die Apparatekosten auch bei der Hochtemperaturfahrweise niedrig.

Da das bei Niedertemperaturfahrweise erzeugte EDC ohne zusätzliche Reinigungsschritte bereits Spaltqualität aufweist, kann Energie zur destillativen Reinigung des erzeugten EDC's eingespart werden.

Die Reaktionsenthalpie der Ethylenchlorierung kann nahezu quantitativ, in Form von Sattdampf mit einer technisch wie wirtschaftlich interessanten Dampfspannung, zurückgewonnen werden; bei der Hochtemperaturfahrweise mit zusätzlich reduziertem Energieverbrauch für die destillative Reinigung des erzeugten EDC's, da dieses frei von Leichtsiedern ist.

Mit der erfindungsgemäßen Verfahrensweise erhält man größtmögliche Flexibilität zwischen Direkt- und Oxichlorierung von Ethylen, entsprechend den jeweils herrschenden Randbedingungen, bei optimaler Energieausnutzung bzw. Energieersparnis im Rahmen des Vinylchlorid-Herstellungsprozesses.

Überraschenderweise hat sich auch gezeigt, daß das erfindungsgemäße Katalysatorsystem allen bisher bekannten Katalysatorgemischen zur Direktchlorierung von Ethylen, sowohl hinsichtlich Selektivität, als auch bezüglich Korrosionsverhalten deutlich überlegen ist, wobei diese Überlegenheit vor allem erst bei erhöhten Reaktionstemperaturen ganz klar zu Tage tritt. So bildet sich auch bei der Hochtemperaturfahrweise praktisch kein Ethylchlorid und auch signifikant weniger 1,1,2-Trichlorethan als beispielsweise die DE-A 3148450 bzw. die EP-PS 111203 lehren. Aufgrund dessen, daß das erfindungsgemäße Katalysatorsystem sowohl bei Nieder- wie Hochtemperaturfahrweise weitaus weniger korrosiv wirkt als vergleichbare Katalysatorgemische gemäß dem Stand der Technik, ist ersichtlich, daß das erfindungsgemäße Katalysatorsystem überraschenderweise

thermisch sehr stabil sein muß.

Die nachfolgenden Beispiele dienen der weiteren Erläuterung der Erfindung:

## Beispiel 1

Es wurden Korrosionsversuche mit verschiedenen Katalysatorgemischen bei 84°C (Siedetemperatur von EDC) und bei 212°C (Siedetemperatur von Hexachlorbutadien) durchgeführt. Dazu wurden jeweils V4A-Edelstahlplättchen mit den Maßen 40 x 20 x 2 mm in flüssiges EDC bzw. Hexachlorbutadien eingetaucht. In der Flüssigkeit befanden sich jeweils 5000 Gewppm Eisen-III-Chlorid bzw. ein äquimolares Gemisch aus Ammoniumchlorid und Eisen-III-Chlorid bzw. ein äquimolares Gemisch aus Natriumchlorid und Eisen-III-Chlorid bzw. ein äquimolares Gemisch aus ortho-Kresol und Eisen-III-Chlorid bzw. ein äquimolares Gemisch aus Natriumchlorid, ortho-Kresol und Eisen-III-Chlorid, jeweils in einer Konzentration von 5000 Gewppm, berechnet als Eisen-III-Chlorid, in gelöster bzw. in suspendierter Form. Nach jeweils 24stündigem Rückflußkochen unter gleichzeitigem Einleiten von trockenem Chlorwasserstoffgas wurden die Korrosionsraten durch Differenzwägung der Plättchen gravimetrisch ermittelt. Die Ergebnisse sind in Tabelle 1 zusammengestellt.

Tabelle 1

| Katalysatorsystem | Gewichtsabnahme in $mg/mm^2 \cdot a$ bei 84°C | bei 212°C |
|---|---|---|
| $FeCl_3$ bzw. $HFeCl_4$ | 0,123 | 19,630 |
| $NH_4FeCl_4$ | 0,062 | 9,985 |
| $NaFeCl_4$ | 0,055 | 8,835 |
| $H\text{-}FeCl_3 \cdot$o-Kresolat | 0,034 | 2,803 |
| $Na\text{-}FeCl_3 \cdot$o-Kresolat | 0,025 | 2,020 |

Die Ergebnisse verdeutlichen die Überlegenheit des erfindungsgemäßen Katalysatorsystems hinsichtlich Korrosionsverhalten gegenüber dem Stand der Technik. So entspricht eine Korrosionsrate von 2 $mg/mm^2 \cdot a$ beispielsweise in etwa einem Korrosionsabtrag von 0,25 mm/a. Ein Korrosionsabtrag von < 1 mm/a besagt im allgemeinen, daß keine Werkstoffprobleme hinsichtlich Standzeit vorliegen. Für die Katalysatorsysteme Ammonium- bzw. Natriumtetrachloroferrat errechnen sich demgegenüber bei 212°C Korrosionsabträge von > 1 mm/a, was besagt, daß hier Werkstoffprobleme hinsichtlich Standzeit existieren. Diese Ergebnisse sind umso überraschender, wenn man den erfindungsgemäßen Katalysatorkomplex $H\text{-}FeCl_3$-o-Kresolat für sich allein betrachtet. Jedem Fachmann wäre es eher geläufig gewesen, daß mit erhöhter Temperatur auch die Protonolyse dieses Komplexes vermehrt eintritt und somit die Korrosionsrate zunimmt. Das Gegenteil davon ist jedoch eingetreten, was die Versuchsergebnisse eindeutig untermauern.

## Beispiel 2

Herstellung des Katalysatorsystems $NaFeCl_3$-o-Kresolat: 5 g (31 mmol) Eisen-III-Chlorid wasserfrei wurden in einem Glaskolben in 500 $cm^3$ EDC gelöst bzw. suspendiert und mit 1,8 g (31 mmol) Kochsalz sowie 3,3 g (31 mmol) o-Kresol versetzt. Danach wurde Rückfluß gekocht. Der sich bildende Chlorwasserstoff wurde mittels eines kleinen Stickstoffstromes, der durch den Glaskolben geleitet wurde, ausgetragen und in Wasser absorbiert. Nach 15 Stunden waren 0,7 l Chlorwasserstoff, ermittelt durch Titration des Waschwassers, freigesetzt, d. h. der Komplex

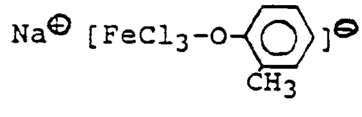

war entstanden.

Anschließend wurde solange bei rund 50 bis 60°C Chlorgas eingeleitet bis die Chlorwasserstoffentwicklung aufhörte. 3 l Chlorwasserstoff wurden freigesetzt, d. h. die Kernchlorierung des Komplexes war vollständig eingetreten:

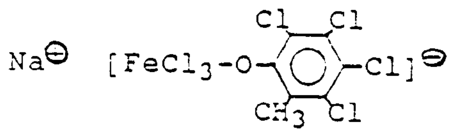

Beispiel 3

Der senkrechtstehende gläserne Reaktionsturm (Innendurchmesser 50 mm, Höhe 300 mm) war mit 2 mm Raschigringen gefüllt und von einem Doppelmantel umgeben, in dem thermostatisiertes Wasser mit einer Temperatur von 84°C umgewälzt wurde. Diese Zusatzheizung war trotz der an sich exotherm verlaufenden Reaktion zwischen Chlor und Ethylen zur Deckung des Wärmeverlustes durch Abstrahlung (ungünstiges Verhältnis Oberfläche zu Wärmefreisetzung) erforderlich, damit EDC aus dem Reaktor abdestillierte. Über eine Dosierpumpe wurde Reaktionsflüssigkeit mit einer Geschwindigkeit von 0,6 cm/s in einer Menge von 40 l/h über den mit Glas-Raschigringen gefüllten Reaktor im Kreis umgewälzt. In das umlaufende Reaktionsmedium wurden 40 Nl/h Chlor und 250 $Ncm^3/h$ Sauerstoff eingeleitet, während 40,2 Nl/h Ethylen über eine im Reaktionsturm befindliche Glasfritte von unten in den Reaktor eingeblasen wurden.

Der Reaktor wurde jeweils mit 500 $cm^3$ EDC beschickt, in dem gemäß Beispiel 2 hergestellte unterschiedliche Katalysatorsysteme in einer Konzentration von 0,5 Gew.% berechnet als dasjenige Metallchlorid, welches das Zentralatom des jeweiligen Katalysatorkomplexes darstellt, gelöst bzw. suspendiert vorlagen. Das abdestillierte EDC wurde in einem Wasserkühler kondensiert und gesammelt. 175 g/h erzeugtes EDC wurden mit Hilfe eines Kondensatteilers abgezweigt und entnommen, während überschüssiges Kondensat in die Reaktionszone zurückgeleitet wurde. Mittels einer Kühlfalle wurden ein weiterer EDC-Anteil aus dem Abgasstrom - im wesentlichen Sauerstoff, überschüssiges Ethylen und Stickstoff, welcher zwecks Inertisierung in einer Menge von 2 Nl/h nach dem Kondensator zugespeist wurden, abgeschieden. Das vereinte erzeugte EDC wurde gaschromatographisch auf Reinheit unsersucht. Das Abgas aus der Kältefalle wurde ebenfalls gaschromatographisch auf leichtsiedende Nebenprodukte und Chlor untersucht. Chlor wurde in keinem der Versuche gefunden, d. h. der Umsatz war immer quantitativ.

Folgende Katalysatorsysteme wurden untersucht:

A) $HFeCl_3$-m-Kresolat, hergestellt durch Zugabe von äquimolaren Mengen an Eisen-III-Chlorid und m-Kresol

B) $NaFeCl_3$-o-Kresolat, hergestellt durch Zugabe von äquimolaren Mengen an NaCl, $FeCl_3$ und o-Kresol

C) Ca[$FeCl_3$-p-Kresolat]$_2$ hergestellt durch Zugabe äquivalenter Mengen an $CaCl_2$ und an dem äquimolaren Gemisch von $FeCl_3$ und p-Kresol

D) $NaFeCl_4$-Phenolat, hergestellt durch Zugabe äquimolarer Mengen an NaCl, $FeCl_4$ und Phenol

E) $NaBiCl_3$-Resorcinat, hergestellt durch Zugabe äquimolarer Mengen an NaCl, $BiCl_3$ und Resorcin

F) Ce[$AlCl_3$-o-Kresolat]$_3$, hergestellt durch Zugabe äquivalenter Mengen an $CeCl_3$ und an dem äquimolaren Gemisch aus $AlCl_3$ und o-Kresol

G) $HFeCl_4$, hergestellt durch Zugabe von $FeCl_3$, HCl entsteht bei der Reaktion

H) $NH_4FeCl_4$, hergestellt durch Zugabe äquimolarer Mengen an $NH_4Cl$ und $FeCl_3$

I) $KFeCl_4$, hergestellt durch Zugabe äquimolarer Mengen an KCl und $FeCl_3$

Die Ergebnisse sind in Tabelle 2 festgehalten. Die Versuchsdauer betrug jeweils 24 Stunden

Tabelle 2

| Katalysatorsystem | Destillat Ethylchlorid | 1,1,2-Trichlorethan | Reaktionsmedium 1,1,2-Trichlorethan |
|---|---|---|---|
| A | <1 Gewppm | 550 Gewppm | 0,09 Gew.% |
| B | <1 Gewppm | 290 Gewppm | 0.06 Gew.% |
| C | <1 Gewppm | 310 Gewppm | 0,08 Gew.% |
| D | <1 Gewppm | 350 Gewppm | 0,08 Gew.% |
| E | <1 Gewppm | 380 Gewppm | 0,075 Gew.% |
| F | <1 Gewppm | 480 Gewppm | 0,086 Gew.% |
| G | 650 Gewppm | 2500 Gewppm | 0,53 Gew.% |
| H | 18 Gewppm | 850 Gewppm | 0,10 Gew.% |
| I | 25 Gewppm | 1430 Gewppm | 0,22 Gew.% |

Die Ergebnisse der Versuche A bis F zeigen deutlich die Überlegenheit der erfindungsgemäßen Katalysatorsysteme hinsichtlich Selektivität gegenüber dem Stand der Technik (Versuche G bis I). Vor allem das Fehlen von Ethylchlorid ist von Bedeutung, weil Ethylchlorid bei der EDC-Pyrolyse bekanntlich Butadien bildet.

Beispiel 4

Es wurde analog Beispiel 3 gearbeitet. Folgende Katalysatorsysteme wurden durch Zusammenmischen in der Versuchsapparatur in situ hergestellt und zwar in einer Konzentration von jeweils 0,4 Gew.%, berechnet als $FeCl_3$:

A) 1 Mol $FeCl_3$ und 0,9 Mol o-Kresol
B) 1 Mol $FeCl_3$ und 1,1 Mol o-Kresol
C) 1 Mol $FeCl_3$ und 0,5 Mol o-Kresol
D) 1 Mol $FeCl_3$ und 2 Mol o-Kresol
E) 1,5 Mol NaCl, 1 Mol $FeCl_3$ und 0,9 Mol o-Kresol
F) 0,5 Mol Nacl, 1 Mol $FeCl_3$ und 1,1 Mol o-Kresol

Die Ergebnisse sind in Tabelle 3 aufgelistet.

Tabelle 3

| Katalysasystem | Destillat-Zusammensetzung Ethylchlorid | 1,1,2-Trichlorethan |
|---|---|---|
| A | <1 Gewppm | 530 Gewppm |
| B | <1 Gewppm | 560 Gewppm |
| C | 480 Gewpmm | 2300 Gewppm |
| D | 530 Gewppm | 2150 Gewppm |
| E | <1 Gewppm | 610 Gewppm |
| F | <1 Gewppm | 520 Gewppm |

Aus den Ergebnissen ist ersichtlich, daß ein molares Verhältnis von $FeCl_3$ zu o-Kresol mit einem Streubereich von 0,9 bis 1,1 : 1 für die Selektivität wichtig, aber der Alkali- bzw. Erdalkalichlorid-Gehalt auch außerhalb der Mengenäquivalenz zu $FeCl_3$ wenig kritisch ist. Trotzdem ist man bemüht, die Mengenäquivalenz der Alkali- bzw. Erdalkalichloride möglichst einzuhalten, weil die von der jeweiligen Äquivalenz nach oben abweichenden Mengen als inertes Material starke Errosionsprobleme geben können.

Beispiel 5

Gemäß Figur 1 wurden, mit Hilfe der Kreislaufpumpe P1, über Leitung 5, den leeren Schleifenreaktor R, die Pumpenvorlage V und den Wasserkühler K1 50 $m^3$/h EDC im Kreis umgewälzt. Das Kreislaufsystem wurde über Leitung 4 mit äquimolaren Mengen von $FeCl_3$, NaCl und o-Kresol in einer Konzentration von 0,5 Gew.%, berechnet als $FeCl_3$ und auf die Menge an umlaufendem EDC bezogen, beschickt.
Bei 70°C Reaktortemperatur und einem Druck von 3 bar absolut wurde Chlor mit Ethylen zur Reaktion gebracht, in dem über Leitung 1 45,1 $Nm^3$/h Ethylen, über Leitung 2 45 $Nm^3$/h verdampftes Flüssigchlor und über Leitung 3 500 Nl/h Sauerstoff zugespeist wurden.
Der Durchmesser der Reaktionsanlage betrug 100 mm und die Länge des Reaktionsrohres lag bei 16000 mm. Ein Teil der freigesetzten Reaktionswärme wurde zur Erzeugung von EDC-Dampf in der Pumpenvorlage V, wo ein Druck von etwa 0,67 bar absolut herrschte, die restliche Reaktionswärme wurde im Kühler K1 ins Kühlwasser abgeführt, um die Reaktionstemperatur im Reaktor R bei 70°C konstant zu halten. Der Druck wurde im Reaktionsteil über einen Druckregler PC konstant bei 3 bar absolut eingestellt. Über Leitung 6 verdampften bei einer Temperatur von 70°C 250 kg/h EDC in den Sumpf der Rektifizierkolonne F, die mit einer 2000 mm hohen Packung, bestehend aus 1/2 Zoll Keramikringen, gefüllt war. Über Leitung 7 floß,in Abhängigkeit vom Stand in der Pumpenvorlage V, Sumpfprodukt der Rektifizierkolonne F. Die am Kopf der Rektifizierkolonne in einer Menge von 650 kg/h austretenden Brüden strömten über Leitung 8 in den Kondensator K2 und wurden kondensiert. Das dabei anfallende Kondensat floß über Leitung 9 in den Sammler S und wurde von dort über Leitung 10 und 11 mit Hilfe der Pumpe P2 in einer Menge von 400 kg/h, über einen Mengenregler (flow controller) FC festwertgeregelt, als flüssiger Rücklauf der Kolonne F aufgegeben, während der Rest des Destillates (etwa 51,5 kg/h), in Abhängigkeit vom Stand im Sammler S, geregelt über Leitung 12, in das Kreislaufsystem 5 gepumpt wurde.
Im Sumpf der Rektifizierkolonne F wurde das erzeugte EDC über Leitung 19 in einer Menge von 198,5 kg/h standgeregelt entnommen.
Die nicht kondensierbaren Bestandteile, im wesentlichen Ethylen, Sauerstoff und Stickstoff, der am Gasaustrittsstutzen des Sammlers S zugespeist wurde (in der Zeichnung nicht enthalten), strömten über Leitung 13, über PC auf einen Druck von 0,66 bar absolut geregelt, in die mit EDC als Betriebsmedium arbeitende Flüssigkeitsringpumpe P3, die den erforderlichen Unterdruck von etwa 0,66 bar absolut gemessen in der Gasleitung 13, erzeugte. Über Leitung 14 entwich das Abgas bei 18 in die Atmosphäre bzw. in eine Verbrennungsanlage.
Frisch-EDC konnte über Leitung 20 zugeführt werden. Gebrauchtes EDC, mit einem geringen Gehalt an Gasen beladen, wurde über Leitung 15 in den mit Wasser gekühlten Behälter B, der über Leitung 17 in die Abgasleitung 14 beatmet war, gepuffert und über Leitung 16 standgeregelt ins Reaktionssystem abgegeben.
Das erzeugte EDC hatte folgende Qualität:

| | |
|---|---|
| Ethylchlorid | < 1 Gewppm |
| 1,1,2-Trichlorethan | 120 Gewppm |
| 1,2-Dichlorethan | 99,98 Gewppm |

Dieses EDC konnte ohne weitere Reinigung direkt in einem Spaltofen zu Vinylchlorid und Chlorwasserstoff umgesetzt werden, ohne daß die Spaltkinetik oder die Nebenprodukt- und Koksbildung in irgendeiner Weise beeinträchtigt wurde, wie ein Betriebsversuch, nach vorherigem Ansammeln entsprechener Mengen, dieses nach dem erfindungsgemäßen Verfahren hergestellten EDC's, bewiesen hatte. Selbst nach 4monatigem Versuch war weder eine Qualitätseinbuße des erzeugten EDC's noch ein Katalysatorverlust zu beklagen. Eine Hochsiederanreicherung im Reaktorkreislauf war auch nicht zu beobachten.

Beispiel 6

Der Reaktor R gemäß Figur 2 bestand aus einem senkrecht stehenden Turm (Durchmesser 200 mm, Länge 4000 mm), der mit einer Packung gefüllt war. Über Leitung 23 und dem Wärmetauscher H, der mit Dampf beheizbar war, um die gesamte Apparatur durch Azeotropdestillation mit EDC wasserfrei machen zu können, wurde mittels der Pumpe P1 50 $m^3$/h EDC im Kreis umgewälzt. In diesem Kreislaufstrom befand sich $FeCl_3$, NaCl und m-Kresol in äquimolaren Mengen zueinander und in einer Konzentration von 0.5 Gew%, berechnet

als $FeCl_3$, gelöst bzw. suspendiert. Über Leitung 1 wurden 45,2 $Nm^3/h$ Ethylen, über Leitung 2 45 $Nm^3/h$ Chlor als Flüssigchlor und über Leitung 27 400 Nl/h Sauerstoff zugegeben. Die Reaktion zwischen Ethylen und Chlor wurde bei 165°C und einem Druck von 7,3 bar absolut durchgeführt, wobei die dabei freiwerdende Reaktionswärme gänzlich zur Erzeugung Von EDC-Dampf genutzt wurde. Über Leitung 6 verdampften bei einem Druck von etwa 7,1 bar 1345 kg/h EDC in den Sumpf der Rektifizierkolonne F, die mit einer 2000 mm hohen Packung, bestehend aus 1/2 Zoll Keramikringen, ausgerüstet war. Über Leitung 8 strömten 1545 kg/h EDC-Brüden mit einer Temperatur von etwa 162°C in den Dampferzeuger WT, in dem, unter Kondensation der Brüden, standgeregelt über Leitung 29, in einer Menge von 200 kg/h, gemessen mit dem Durchflußzähler FQ, zugeführtes 150-grädiges Kesselspeisewasser dementsprechend in Sattdampf von einem Druck von 4,9 bar absolut umgewandelt wurde, der nach Phasentrennung in der Dampftrommel DT druckgeregelt über Leitung 32 in das Dampfnetz abgegeben wurde. Bei 26 wurde Stickstoff für Inertisierzwecke aufgegeben. Die kondensierten Brüden floßen über Leitung 28 in den Abscheider A1 und wurden von dort über Leitung 24 mit Hilfe der Pumpe P2, festwertgeregelt über FC, und Leitung 25 mit einer Menge von 200 kg/h als flüssiger Rücklauf der Kolonne F aufgegeben bzw. über Leitung 22 standgeregelt ins Kreislaufsystem gepumpt. Das nicht kondensierbare Abgas strömte über Leitung 30 zum Kühler K, dabei etwaig anfallendes Kondensat wurde im Abscheider A2 gesammelt und floß über Leitung 31 zum Abscheider A1 zurück. Das Abgas wurde druckgeregelt auf 7,05 bar absolut bei 21 ins Freie abgelassen bzw. einer Verbrennungsanlage zugeführt.

Mit Hilfe des Analysators AR wurde das Abgas auf Chlor analysiert. Über Leitung 7 wurde, geregelt vom Stand im Reaktionsturm, Sumpfprodukt von der Kolonne F in das Reaktionssystem abgeführt. Chlor wurde im Analysator AR nicht gefunden, d. h. der Chlorumsatz erfolgte quantitativ.

Über Leitung 19 wurden 198,5 kg/h erzeugtes EDC standgeregelt entnommen. Das erzeugte EDC hatte folgende Zusammensetzung:

| | |
|---|---|
| Ethylchlorid | < 1 Gewppm |
| 1,1,2-Trichlorethan | 3100 Gewppm |
| 1,2-Dichlorethan | 99,68 Gewppm |

Wegen des relativ hohen Gehaltes an 1,1,2-Trichlorethan mußte das Roh-EDC vor seinem Einsatz zur thermischen Spaltung in Vinylchlorid und Chlorwasserstoff noch in bekannter Weise von diesem Hochsieder befreit werden.

Selbst nach 3 Monaten Versuchsdauer war keine Katalysatordesaktivierung bzw. Katalysatorverarmung aufgetreten, d. h. das erfindungsgemäße Katalysatorsystem war thermisch stabil.

## Patentansprüche

1. Katalysatorsystem zur Herstellung von 1,2-Dichlorethan durch Reaktion zwischen Ethylen und Chlor in einem Lösungsmittel, gegebenenfalls in Gegenwart eines Inhibitors zur Verringerung der Nebenproduktbildung, bestehend aus einem Phenolatchlorokomplex der Formel

$$Me^{+n}[Z^{+m}Cl_m \cdot L]_n$$

worin

| | |
|---|---|
| n | eine ganze Zahl von 1 bis 3 ist, |
| m | eine ganze Zahl von 1 bis 6 ist, |
| Cl | ein Chloridanion bedeutet, |
| $Me^+$ | ein Wasserstoffproton und/oder Metallkation der Elemente der 1. und/oder 2. Hauptgruppe oder der Lanthaniden-Gruppe des Periodensystems der Elemente (PSE) bedeutet, |
| $Z^+$ | ein Metallkation der Elemente der 3., 4., 5. oder 6. Hauptgruppe oder der 1., 4., 6. oder 8. Nebengruppe des PSE bedeutet, und |
| L | ein Phenolatanion ist der Formel: |

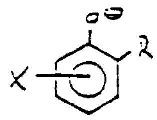 und/oder 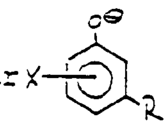 und/oder 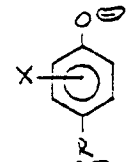 und/oder 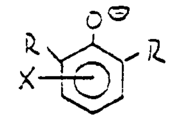

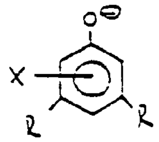 und/oder 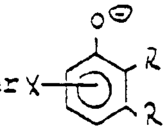 und/oder 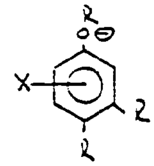 und/oder 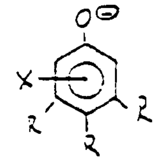

worin

X 0 bis 4 Chloratome bedeutet und

R ein Wasserstoffatom, und/oder eine Hydroxylgruppe, und/oder ein Halogen, und/oder ein linearer oder verzweigter Alkyl- oder Chloralkylrest mit 1 bis 6 C-Atomen, und/oder ein Alkoxy- oder Chloralkoxyrest mit 1 bis 6 C-Atomen in linearer oder verzweigter Anordnung, ist.

2. Katalysatorsystem nach Anspruch 1, dadurch gekennzeichnet, daß das Katalysatorsystem aus Wasserstoff-meta-kresolat-trichloroferrat, Natrium-ortho-kresolat-trichloroferrat, Calcium-bis-para-kresolat-trichloroaluminat oder Cer-tris-ortho-kresolat-trichloroaluminat besteht.

3. Katalysatorsystem nach Anspruch 1, dadurch gekennzeichnet, daß die Herstellung des Katalysatorsystems durch Mischen der Verbindungen $Z^{+m}Cl_m$, $Me^{+n}Cl_n$ und der phenolischen Verbindungen L in äquivalenten Mengen erfolgt, wobei die Äquivalenz zwischen $Z^{+m}Cl_m$ und phenolischer Verbindung L einen Streubereich von jeweils 0.9 bis 1.1 : 1 äquivalenten Anteilen, und die Äquivalenz zwischen $Me^{+n}Cl_n$ und phenolischer Verbindung L einen Streubereich von jeweils 0.5 bis 1.5 : 1 äquivalenten Anteilen aufweist.

4. Katalysatorsystem nach Anspruch 1 oder 3, dadurch gekennzeichnet, daß die Äquivalenz zwischen $Me^{+n}Cl_n$ und phenolischer Verbindung L einen Streubereich von jeweils 0.9 bis 1.1 : 1 äquivalenten Anteilen aufweist.

5. Katalysatorsystem nach Anspruch 1, 2, 3 oder 4, dadurch gekennzeichnet, daß die Herstellung durch Mischen von Chlorwasserstoff, meta-Kresol und Eisen-III-Chlorid erfolgt.

6. Katalysatorsystem nach Anspruch 1, 2, 3 oder 4, dadurch gekennzeichnet, daß die Herstellung durch Mischen von Natriumchlorid, ortho-Kresol und Eisen-III-Chlorid erfolgt.

7. Katalysatorsystem nach Anspruch 1, 2, 3 oder 4, dadurch gekennzeichnet, daß die Herstellung durch Mischen von Calciumchlorid, para-Kresol und Aluminiumchlorid erfolgt.

8. Verfahren zur Herstellung von 1,2-Dichlorethan durch Reaktion von Ethylen mit Chlor, in einer Reaktionszone, die ein im Kreis umlaufendes flüssiges Reaktionsmedium, bestehend aus Chlorkohlenwasserstoffen mit 2 Kohlenstoffatomen, enthält, bei einer Temperatur unterhalb der Verdampfungstemperatur des Mediums, bei dem in der Reaktionszone herrschendem Druck, gegebenfalls in Gegenwart von Sauerstoff als Inhibitor zur Vermeidung von Nebenreaktionen, in Gegenwart eines Katalysatorsystems gemäß den obigen Ansprüchen, dadurch gekennzeichnet, daß

a) die Konzentration des im Reaktionsmedium gelösten oder suspendierten Katalysatorsystems $Me^{+n}[Z^{+m}Cl_m \cdot L]_n$, berechnet als $Z^{+m}Cl_m$ 0.01 bis 1.0 Gew%, bezogen auf die Menge an Reaktionsmedium, beträgt und das Katalysatorsystem, unter Ersatz des verbrauchten Ethylen und Chlor im Kreis geführt wird, wobei man

b) bei Reaktionstemperaturen von 0 bis 300°C und bei Drükken, die ein Sieden des Reaktionsmediums im Reaktionsraum verhindern, unter teilweiser bis vollständiger Ausnutzung der bei der Ethylenchlorierung freiwerdenden Reaktionsenthalpie, zur Erzeugung von 1,2-Dichlorethan-Dampf, dampfförmiges 1,2-Dichlorethan aus der Reaktionszone in eine Zone niedrigeren Druckes abzieht und diese Dämpfe dabei in den Sumpf einer Rektifizierkolonne einleitet, und

c) bei Reaktionstemperaturen von 0 bis 120°C, die Brüden am Kolonnenkopf durch Kühlen mit Wasser oder Luft kondensiert, bei Reaktionstemperaturen von 120 bis 300°C die Kondensationswärme der Brüden, durch Wärmetausch der Brüden der Rektifizierkolonne mit einem Wärmetauschmedium ausnutzt, und

d) unter Aufrechterhaltung eines Mindestrücklaufverhältnisses, ausgedrückt als Gewichtsverhältnis Rücklauf zu erzeugtem Produkt, bei der Niedertemperaturfahrweise von 2:1 Gewichtsteilen und bei der Hochtemperaturfahrweise von 1:1 Gewichtsteilen, die kondensierten Brüden bei der Niedertemperaturfahrweise mehr oder weniger stark unterkühlt, bei der Hochtemperaturfahrweise jedoch um maximal 5°C unterkühlt, bezogen auf die Siede- oder Kondensationstemperatur, in den Reaktionsraum zurückführt, und

e) im Sumpf der Rektifizierkolonne das erzeugte 1,2-Dichlorethan in flüssiger Form entnimmt.

**9.** Verfahren nach Anspruch 8, dadurch gekennzeichnet, daß die Reaktionstemperatur 40 bis 80°C beträgt, der Druck im Reaktionsraum zwischen 1 und 5 bar absolut beträgt und die Brüden am Kopf der Rektifizierkolonne bei einem Druck von 0.2 bis 0.9 bar abs. durch indirekten Wärmetausch mit Wasser oder Luft kondensiert werden und das in den Reaktionsraum zurückgeführte Brüdenkondensat eine gegenüber der Siedetemperatur um etwa 5 bis 50° verminderte Temperatur aufweist.

**10.** Verfahren nach Anspruch 8, dadurch gekennzeichnet, daß die Reaktionstemperatur 140 bis 170°C beträgt, der Druck im Reaktionsraum zwischen 5 und 10 bar absolut beträgt und die Brüden am Kopf der Rektifizierkolonne bei einem Druck von 4.5 bis 8.3 bar abs. durch indirekten Wärmetausch mit heißem Wasser, unter Erzeugung von Sattdampf mit einem Druck von 2.25 bis 6.0 bar abs., kondensiert werden.

**11.** Verfahren nach Anspruch 8, 9 oder 10, dadurch gekennzeichnet, daß als Reaktionsmedium 1,2-Dichlorethan mit einer Geschwindigkeit von 0.1 bis 5 m/s im Kreis umgewälzt wird.

**12.** Verfahren nach Anspruch 8, 9, 10 oder 11, dadurch gekennzeichnet, daß das molare Verhältnis von Ethylen zu Chlor zwischen 1 : 1 und 1.005 : 1 beträgt und die Gasgeschwindigkeit im Reaktionsraum zwischen 0.01 und 1 m/s, bezogen auf den Ethylengasstrom, beträgt.

**13.** Verfahren nach Anspruch 8, 9, 10, 11 oder 12, dadurch gekennzeichnet, daß die Verweilzeit des Reaktandengemisches bei 0.2 bis 2 Minuten liegt, bezogen auf den leeren Reaktions-, Misch- und Umwälzraum bei Normalbedingungen (0°C, 1013 mbar), und die Reaktionszeit der Reaktanden im Reaktionsraum zwischen 1.5 und 60 Sekunden beträgt.

**14.** Verfahren nach Anspruch 8, 9, 10, 11, 12, oder 13, dadurch gekennzeichnet, daß als Inhibitor Sauerstoff oder Luft in einer Konzentration von 0.01 bis 10 Vol%, jeweils als Sauerstoff berechnet und auf die Chlorgasmenge bezogen, eingesetzt werden.

## Claims

**1.** Catalyst system for the preparation of 1,2-dichloroethane by reacting ethylene and chlorine in a solvent, if desired in the presence of an inhibitor for reducing the formation of byproducts, the catalyst system comprising a phenolate/chlorine complex of the formula

$$Me^{+n}[Z^{+m}Cl_m \cdot L]_n$$

in which

n is an integer from 1 to 3,
m is an integer from 1 to 6,
Cl is a chloride anion,
$Me^+$ is a hydrogen proton and/or a metal cation of elements from the 1st and/or 2nd main group or of the lanthanide group of the Periodic Table of the Elements (PTE),
$Z^+$ is a metal cation of elements of the 3rd, 4th, 5th or 6th main group or of the 1st, 4th, 6th or 8th sub-group of the PTE, and
L is a phenolate anion of the formula:

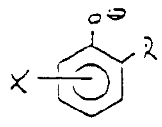 and/or 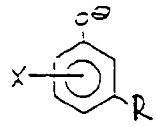 and/or 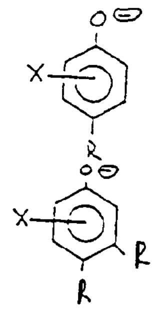 and/or 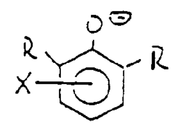

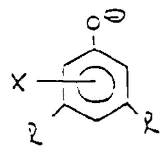 and/or 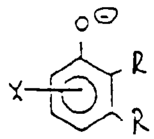 and/or and/or 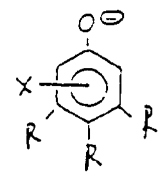

in which

X denotes 0 to 4 chlorine atoms, and

R is a hydrogen atom and/or a hydroxyl group and/or a halogen and/or a linear or branched alkyl or chloroalkyl radical having from 1 to 6 C atoms and/or an alkoxy or chloroalkoxy radical having 1 to 6 C atoms in a linear or branched arrangement.

**2.** Catalyst system according to Claim 1, characterised in that the catalyst system consists of hydrogen meta-cresolate trichloroferrate, sodium ortho-cresolate trichloroferrate, calcium bis-para-cresolate trichloroaluminate or cerium tris-ortho-cresolate trichloroaluminate.

**3.** Catalyst system according to Claim 1, characterised in that the catalyst system is prepared by mixing the compounds $Z^{+m}Cl_m$, $Me^{+n}Cl_n$ and the phenolic compounds L in equivalent amounts, the equivalence between $Z^{+m}Cl_m$ and the phenolic compound L having a scattering range of in each case from 0.9 to 1.1:1 equivalent proportions, and the equivalence between $Me^{+n}Cl_n$ and the phenolic compounds L having a scattering range of in each case from 0.5 to 1.5 : 1 equivalent proportions.

**4.** Catalyst system according to Claim 1 or 3, characterised in that the equivalence between $Me^{+n}Cl_n$ and the phenolic compound L has a scattering range of in each case from 0.9 to 1.1:1 equivalent proportions.

**5.** Catalyst system according to Claim 1, 2, 3 or 4, characterised in that the preparation is carried out by mixing hydrogen chloride, meta-cresol and iron(III) chloride.

**6.** Catalyst system according to Claim 1, 2, 3 or 4, characterised in that the preparation is carried out by mixing sodium chloride, ortho-cresol and iron(III) chloride.

**7.** Catalyst system according to Claim 1, 2, 3 or 4, characterised in that the preparation is carried out by mixing calcium chloride, para-cresol and aluminium chloride.

**8.** Process for the preparation of 1,2-dichloroethane by reacting ethylene with chlorine, in a reaction zone which contains a circulating liquid reaction medium comprising chlorinated hydrocarbons having 2 carbon atoms, at a temperature below the evaporation temperature of the medium, at the pressure prevailing in the reaction zone, if desired in the presence of oxygen as an inhibitor for preventing side reactions, in the presence of a catalyst system according to the above claims, characterised in that

a) the concentration of the catalyst system $Me^{+n}[Z^{+m}Cl_m \cdot L]_n$, dissolved or suspended in the reaction medium is, calculated as $Z^{+m}Cl_m$, from 0.01 to 1.0 % by weight, based on the amount of reaction medium, and the catalyst system is circulated with replenishment of the consumed ethylene and chlorine, where,

b) at reaction temperatures of from 0 to 300°C and at pressures which prevent the reaction medium boiling in the reaction space, with utilisation of all or some of the reaction enthalpy liberated during the chlorination of ethylene to generate 1,2-dichloroethane vapour, vapour-form 1,2-dichloroethane is removed from the reaction zone into a zone of lower pressure, and these vapours are introduced into the bottom of a rectification column, and,

c) at reaction temperatures of from 0 to 120°C, the vapours at the head of the column are condensed by cooling with water or air, at reaction temperatures of from 120 to 300°C, the heat of condensation of the vapours is utilised by heat exchange of the vapours from the rectification column with a heat-exchange medium, and,

d) while maintaining a minimum reflux ratio, expressed as the ratio by weight of the reflux to the product generated, of 2:1 parts by weight in the low-temperature procedure and of 1:1 parts by weight in the high-temperature procedure, the condensed vapours are fed back into the reaction space after more

or less considerable cooling in the low-temperature procedure, but after cooling by a maximum of 5°C in the high-temperature procedure, based on the boiling or condensation temperature, and
e) the 1,2-dichloroethane produced is removed in liquid form from the bottom of the rectification column.

**9.** Process according to Claim 8, characterised in that the reaction temperature is from 40 to 80°C, the pressure in the reaction space is between 1 and 5 bar absolute, and the vapours at the head of the rectification column are condensed at a pressure of from 0.2 to 0.9 bar abs. by indirect heat exchange with water or air, and the vapour condensate fed back into the reaction space has a temperature of from about 5 to 50°C below the boiling temperature.

**10.** Process according to Claim 8, characterised in that the reaction temperature is from 140 to 170°C, the pressure in the reaction space is between 5 and 10 bar absolute, and the vapours at the head of the rectification column are condensed at a pressure of from 4.5 to 8.3 bar abs. by indirect heat exchange with hot water, with generation of saturated steam at a pressure of from 2.25 to 6.0 bar abs.

**11.** Process according to Claim 8, 9 or 10, characterised in that the reaction medium circulated is 1,2-dichloroethane at a speed of from 0.1 to 5 m/s.

**12.** Process according to Claim 8, 9, 10 or 11, characterised in that the molar ratio between ethylene and chlorine is between 1:1 and 1.005:1, and the gas flow rate in the reaction space is between 0.01 and 1 m/s, based on the ethylene gas stream.

**13.** Process according to Claim 8, 9, 10, 11 or 12, characterised in that the residence time of the reactant mixture is from 0.2 to 2 minutes, based on the empty reaction, mixing and circulation space under standard conditions (0°C, 1013 mbar), and the reaction time of the reactants in the reaction space is between 1.5 and 60 seconds.

**14.** Process according to Claim 8, 9, 10, 11, 12 or 13, characterised in that the inhibitor employed is oxygen or air in a concentration of from 0.01 to 10 % by volume, in each case calculated as oxygen and based on the amount of chlorine gas.

## Revendications

**1.** Système de catalyseurs pour la préparation du 1,2-dichloréthane par réaction entre l'éthylène et le chlore dans un solvant, le cas échéant en présence d'un inhibiteur, pour réduire la formation de sous-produits, constitué d'un complexe phénolate-chlore répondant à la formule :

$$Me^{+n}[Z^{+m}Cl_m \cdot L]_n$$

dans laquelle

n  est un nombre entier de 1 à 3,
m  est un nombre entier de 1 à 6,
Cl  est un anion chlorure,
$Me^+$  est un proton hydrogène et/ou un cation métallique des éléments des groupes principaux 1 et/ou 2 ou du groupe des lanthanides de la Classification Périodique des Eléments,
$Z^+$  désigne un cation de métal des éléments des groupes principaux 3, 4, 5 ou 6 ou des sous-groupes 1, 4, 6 ou 8 de la Classification Périodique, et
L  est un anion phénolate répondant aux formules :

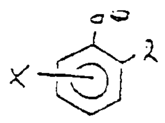 et/ou 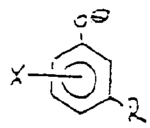 et/ou 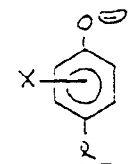 et/ou 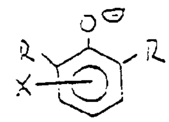

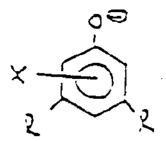 et/ou 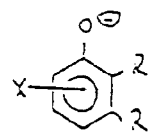 et/ou 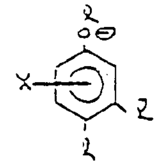 et/ou 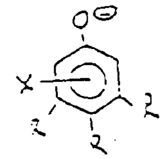

dans lesquelles

X désigne 0 à 4 atomes de chlore et

R est un atome d'hydrogène et/ou un groupe hydroxyle et/ou un atome d'halogène et/ou un radical alkyle ou chloralkyle linéaire ou ramifié en $C_1$-$C_6$, et/ou un radical alcoxy ou chloralcoxy en $C_1$-$C_6$, dans une disposition linéaire ou ramifiée.

2. Système de catalyseurs selon la revendication 1, caractérisé en ce que le catalyseur se compose d'hydrogéno-métacrésolate-trichloroferrate, d'ortho-crésolate-trichloroferrate de sodium, de bis-para-crésolate-trichloraluminate de calcium ou de tris-ortho-crésolate-trichloraluminate de cérium.

3. Système de catalyseurs selon la revendication 1, caractérisé en ce que la préparation du système de catalyseurs s'effectue par mélange des composés $Z^{+m}Cl_m$, $Me^{+n}Cl_n$ et des composés phénoli.ques L dans des quantités équivalentes, l'équivalence entre $Z^{+m}Cl_m$ et le composé phénolique L présentant un domaine de dispersion qui est à chaque fois de 0,9 à 1,1:1 parties équivalentes, et l'équivalence entre $Me^{+n}Cl_n$ et le composé phénolique L un domaine de dispersion qui est à chaque fois de 0,5 à 1,5:1 partie équivalente.

4. Système de catalyseurs selon les revendications 1 ou 3, caractérisé en ce que l'équivalence entre $Me^{+n}Cl_n$ et le composé phénolique L présente un domaine de dispersion qui est à chaque fois de 0,9 à 1,1:1 partie équivalente.

5. Système de catalyseurs selon les revendications 1, 2, 3 ou 4, caractérisé en ce que la préparation s'effectue par mélange d'acide chlorhydrique, de méta-crésol et de chlorures ferrique.

6. Système de catalyseurs selon les revendications 1, 2, 3 ou 4, caractérisé en ce que la préparation s'effectue par mélange de chlorure de sodium, d'ortho-crésol et de chlorure ferrique.

7. Système de catalyseurs selon les revendications 1, 2, 3 ou 4, caractérisé en ce que la préparation s'effectue par mélange de chlorure de calcium, de para-crésol et de chlorure d'aluminium.

8. Procédé de préparation du 1,2-dichloréthane par réaction de l'éthylène avec le chlore, dans une zone réactionnelle qui contient un milieu réactionnel liquide circulant cycliquement, constitué d'hydrocarbures chlorés en $C_2$, à une température inférieure à la température de vaporisation du milieu, sous la pression régnant dans la zone réactionnelle, le cas échéant en présence d'oxygène comme inhibiteur pour éviter des réactions secondaires, en présence d'un système de catalyseurs selon les revendications précédentes, caractérisé en ce que :

a) la concentration du système de catalyseur $Me^{+n}[Z^{+m}Cl_m.L]_n$, dissous ou en suspension dans le mélange réactionnel, calculée en $Z^{+m}Cl_m$, est de 0,01 à 1,0 % en poids par rapport à la quantité de mélange réactionnel et en ce que le système de catalyseur est envoyé dans le cycle, avec remplacement de l'éthylène et du chlore consommés,

b) en éliminant de la zone réactionnelle dans une zone de faible pression, à des températures de réaction de 0 à 300 °C et sous des pressions s'opposant à une ébullition du milieu réactionnel dans l'espace réactionnel, avec utilisation partielle à totale de l'enthalpie de réaction libérée lors du chlorage de l'éthylène, pour la production de vapeur de 1,2-dichloréthane, du l,2-dichloréthane sous forme de vapeur, et en envoyant ces vapeurs au bas d'une colonne de rectification, et

c) en condensant à des températures de réaction de 0 à 120 °C les vapeurs chaudes à la tête de colonne par refroidissement avec de l'eau ou de l'air, à des températures de réaction de 120 à 300 °C, en utilisant la chaleur de réaction de vapeurs chaudes par échange de chaleur des vapeurs chaudes de la colonne

de rectification avec un milieu d'échange de chaleur, et

d) en maintenant un rapport de reflux minimum, exprimé par le rapport ponédral du reflux au produit formé, dans le cas d'un mode de conduite à basse température, de 2:1 parties en poids et dans le cas d'un mode de conduite à haute température, de 1:1 partie en poids, en renvoyant les vapeurs chaudes condensées plus ou moins surfondues dans l'espace réactionnel dans le cas du mode de conduite à basse température, mais surrefroidies à un maximum de 5 °C, par rapport à la température d'ébullition ou de condensation dans le cas d'un mode de conduite à haute température, et

e) en prélevant au bas de la colonne de rectification le 1,2-dichloréthane produit sous forme liquide.

**9.** Procédé selon la revendication 8, caractérisé en ce que la température de réaction est de 40 à 80 °C, la pression dans l'espace réactionnel est comprise entre 1 et 5 bars absolus et les vapeurs chaudes en tête de la colonne de rectification sont condensées sous une pression de 0,2 à 0,9 bar absolu par échange de chaleur indirect avec l'eau ou l'air et en ce que le condensat des vapeurs chaudes renvoyées dans l'espace réactionnel présente par rapport à la température d'ébullition une température abaissée d'environ 5 à 50 °C.

**10.** Procédé selon la revendication 8, caractérisé en ce que la température de réaction est de 140 à 170 °C, la pression dans l'espace réactionnel est comprise entre 5 et 10 bars absolus et les vapeurs chaudes en tête de la colonne de rectification sont condensées sous une pression de 4,5 à 8,3 bars absolus par échange de chaleur indirect avec de l'eau chaude, avec production de vapeur saturée sous une pression de 2,25 à 6,0 bars absolus.

**11.** Procédé selon les revendications 8, 9 ou 10, caractérisé en ce que l'on fait circuler cycliquement comme milieu réactionnel du 1,2-dichloréthane avec une vitesse de 0,1 à 5 m/s.

**12.** Procédé selon les revendications 8, 9, 10 ou 11, caractérisé en ce que le rapport molaire de l'éthylène au chlore est de 1:1 et 1,005:1 et en ce que la vitesse des gaz dans l'espace réactionnel est comprise entre 0,01 et 1 m/s par rapport au courant d'éthylène gazeux.

**13.** Procédé selon les revendications 8, 9, 10, 11 ou 12, caractérisé en ce que le temps de séjour du mélange de réactifs est de 0,2 à 2 minutes, rapporté à l'espace de réaction, de mélange et de circulation vide dans les conditions normales (0 °C, 1013 mbars), et en ce que le temps de réaction des réactifs dans l'espace réactionnel est compris entre 1,5 et 60 secondes.

**14.** Procédé selon les revendications 8, 9, 10, 11, 12 ou 13, caractérisé en ce que l'on utilise comme inhibiteur de l'oxygène ou de l'air à une concentration de 0,01 à 10 % en volume, calculée à chaque fois en oxygène et par rapport à la quantité de chlore gazeux.

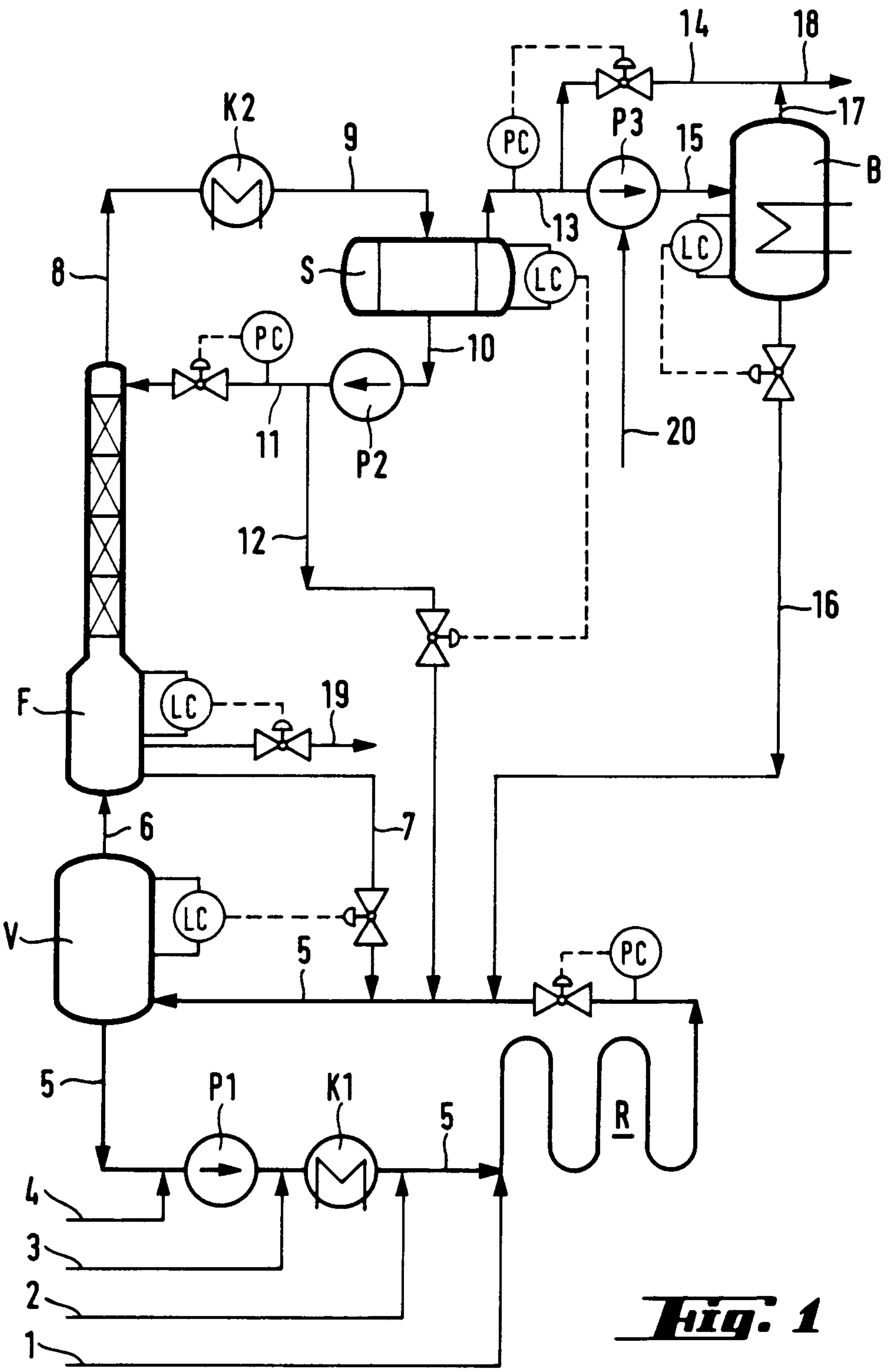
K2
9
14
18
17
PC
P3
15
B
13
S
LC
LC
8
PC
10
11
P2
20
12
16
F
LC
19
6
7
V
LC
5
PC
5
P1
K1
5
R
4
3
2
1

Fig. 1

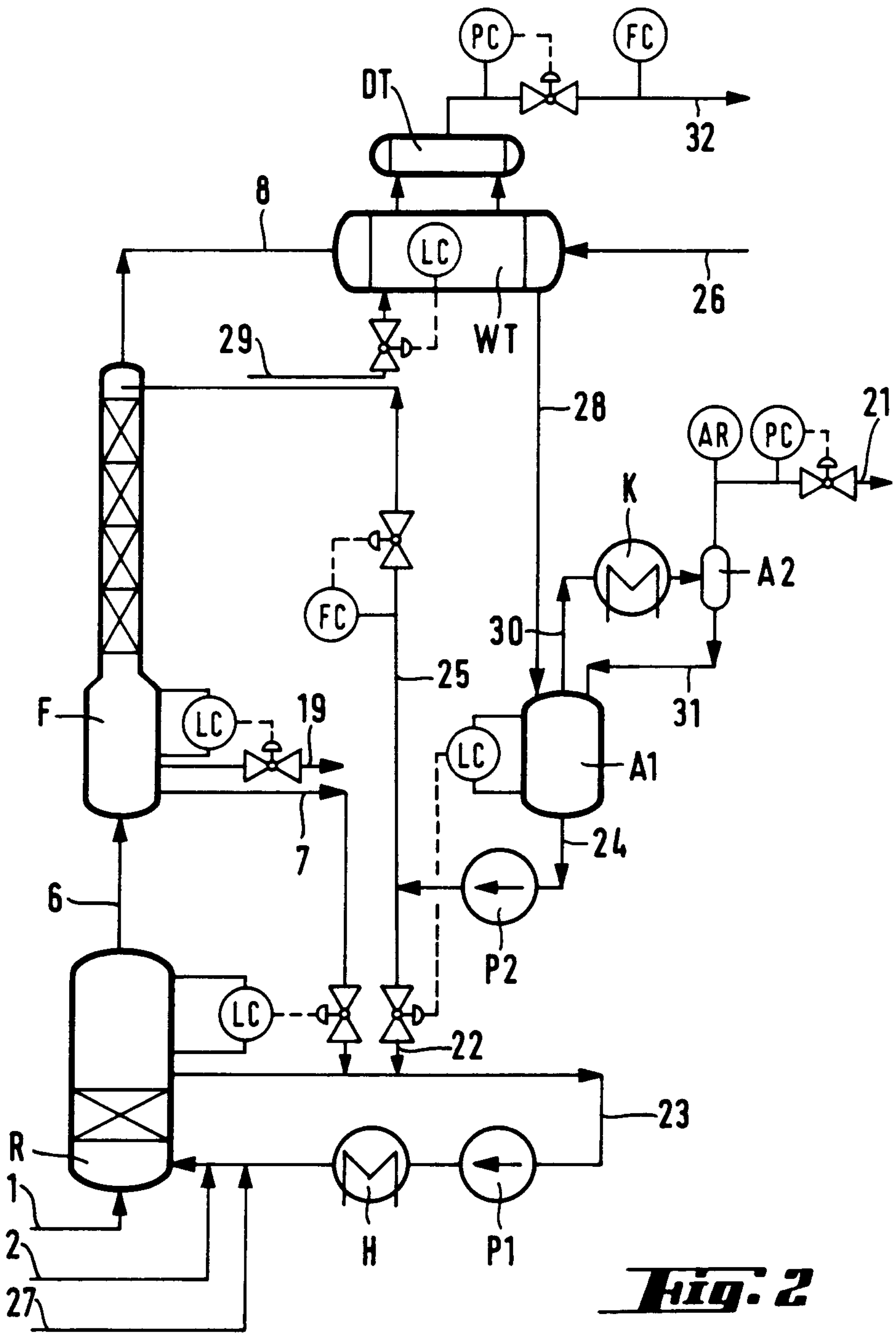
PC
FC
DT
32
8
LC
26
WT
29
28
AR
PC
21
K
A2
FC
30
25
31
F
LC
19
LC
A1
7
24
6
P2
LC
22
23
R
1
2
27
H
P1

Fig. 2